# NEUE EUROPÄISCHE PATENTSCHRIFT

(11) **EP 1 620 377 B2**
(45) Veröffentlichungstag und Bekanntmachung des Hinweises auf die Entscheidung über den Einspruch: **08.03.2017**
(45) Hinweis auf die Patenterteilung: 02.07.2008
(21) Anmeldenummer: 04727249.7
(22) Anmeldetag: 14.04.2004
(51) Int. Cl.: C07C 29/141, C07C 31/20

(54) **VERFAHREN ZUR HYDRIERUNG VON METHYLOLALKANALEN**
METHOD FOR HYDROGENATING METHYLOL ALKANALS
PROCEDE D'HYDROGENATION DE METHYLOL-ALCANALS

(30) Priorität: 16.04.2003 DE 10317543
(43) Veröffentlichungstag der Anmeldung: 01.02.2006
(73) Patentinhaber: BASF SE, 67056 Ludwigshafen am Rhein (DE)
(72) Erfinder: WARTINI, Alexander, 69120 Heidelberg (DE); DERNBACH, Matthias, 69221 Dossenheim (DE); MAAS, Steffen, 55270 Bubenheim (DE)
(86) Internationale Anmeldenummer: PCT/EP2004/003951
(87) Internationale Veröffentlichungsnummer: WO 2004/092097

(56) Entgegenhaltungen:
- EP-A- 0 522 368
- WO-A-92/22521
- DE-A1- 1 957 591

## Beschreibung

Die Erfindung betrifft ein Verfahren zur katalytischen Hydrierung von Methylolalkanalen in der Flüssigphase an einem Hydrierkatalysator bei einem durch Zusatz von tertiärem Amin eingestellten pH-Wert von 6,3 bis 7,8 im Hydrierfeed.

Die katalytische Hydrierung von Carbonylverbindungen wie z.B. Aldehyden zur Herstellung einfacher und funktionalisierter Alkohole nimmt in den Produktionssträngen der chemischen Grundstoffindustrie eine bedeutende Stellung ein. Besonders gilt dies für die Hydrierung von Aldehyden, die über Oxosynthese oder Aldolreaktion zugänglich sind.

Durch Aldolreaktion von Alkanalen mit überschüssigem Formaldehyd in Gegenwart stöchiometrischer Mengen an Base werden Methylolalkanale zugänglich. Aus WO 01/51438 ist es bekannt, anorganische Hydroxide wie Nariumhydroxid oder Calciumhydroxid als Base einzusetzen. WO 98/28253 beschreibt Amine als basische Katalysatoren der Aldolisierung und WO 98/29374 basische Ionentauscher. Das Methylolalkanal wird nach diesen Verfahren als 20 bis 70 Gew.%ige wässrige Lösung erhalten. Der pH-Wert dieser wässrigen Lösung liegt nur bei 3,5 bis 6,0, da der basische Katalysator der Aldolisierung auch die Cannizzaro-Reaktion des Formaldehyds zu Ameisensäure katalysiert, die wiederum die Base zumindestens teilweise neutralisiert.

Will man mehrwertige Alkohole wie Pentaerythrit, Neopentylglykol oder Trimethylolpropan aus wässrigen Methylolalkanal-Lösungen herstellen, müssen diese Lösungen hydriert werden. WO92/22521 betrifft die Herstellung von Neopentylglykol.

Diese Hydrierung wird im allgemeinen bei Temperaturen von über 80°C durchgeführt. Es werden Rückspaltungen der Methylolgruppe zu freiem Aldehyd und darüber hinaus Ether-, Ester- und Acetalbildung im Hydrierreaktor beobachtet. Diese Nebenreaktionen führen zu niedrigen Hydrierselektivitäten und zu niedrigen Ausbeuten an mehrwertigem Alkohol.

Zudem sind viele Hydrierkatalysatoren unter diesen Bedingungen nicht stabil. Insbesondere Katalysatoren auf Basis der Oxide des Aluminiums und Siliziums wie sie aus EP-A 44 444 und WO 95/32171 bekannt sind, verlieren in Gegenwart dieser wässrigen Methylolalkanallösungen unter Hydrierbedingungen durch Austrag von Siliciumdioxid an Härte und werden schlimmstenfalls unbrauchbar.

Der Erfindung lag daher die Aufgabe zugrunde, ein Verfahren zur katalytischen Hydrierung von Methylolalkanalen bereitzustellen, bei dem die Rückspaltung von einmal gebildeten Methylolalkanalen weitgehend unterdrückt, die Bildung von Ethern, Estern und Acetalen weitgehend verhindert und ein positiver Effekt auf die mechanische Stabilität des Katalysators ausgeübt wird. Zudem sollte das Verfahren mehrwertige Alkohole mit guten Hydrierselektivitäten und Ausbeuten zugänglich machen.

Diese Aufgabe wird gelöst durch ein Verfahren zur katalytischen Hydrierung von Methylolalkanalen der allgemeinen Formel 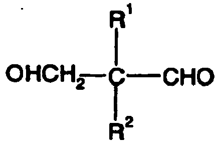 in der R¹ und R² unabhängig voneinander eine weitere Methylolgruppe oder eine Alkylgruppe mit 1 bis 22 C-Atomen oder eine Aryl- oder Aralkylgruppe mit 6 bis 33 C-Atomen bedeuten, in der Flüssigphase an einem Hydrierkatalysator, dadurch gekennzeichnet, dass durch Zusatz mindestens eines tertiären Amins im Hydrierfeed ein pH-Wert von 6,3 bis 7,8 eingestellt wird und dass eine Formaldehyd-arme wässrige Methylolalkanallösung als Hydrierfeed eingesetzt wird, wobei der Gehalt an Formaldehyd unter 5 Gew.-% liegt.

Unter Hydrierfeed wird in dieser Ameldung eine Methylolalkanal der allgemeinen Formel 1 enthaltende wässrige Lösung, insbesondere eine 20 bis 80 Gew.-% Methylolalkanal enthaltende wässrige Lösung, verstanden. Ein solcher Hydrierfeed wird bevorzugt gemäß WO 98/28253 durch Kondensation von Aldehyden mit Formaldehyd hergestellt.

Es wird dabei so verfahren, dass der Aldehyd mit der 2- bis 8-fachen Mengen Formaldehyd in Gegenwart eines tertiären Amins (Aldolisierung) umgesetzt wird, und man das so erhaltene Reaktionsgemisch in zwei Lösungen auftrennt, wobei eine Lösung das erwähnte Methylolalkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Methylolalkanal enthaltende wässrige Lösung kann als Hydrierfeed in dem erfindungsgemäßen Verfahren eingesetzt werden.

Es ist jedoch auch möglich die wässrige Methylolalkanallösung als Hydrierfeed nach anderen Verfahren des Standes der Technik, beispielsweise nach den aus WO 01/51438, WO 97/17313 und WO 98/29374 bekannten Verfahren herzustellen.

Erfindungsgemäß wird eine Formaldehyd-arme wässrige Methylolalkanallösung als Hydrierfeed eingesetzt. In einer Formaldehyd-armen Methylolalkanallösung liegt der Gehalt an Formaldehyd unter 5 Gew.-%. Die Abtrennung von Formaldehyd aus dem Aldolisierungsaustrag, der beispielsweise gemäß WO 98/28253 erhalten wurde, kann nach den aus dem Stand der Technik bekannten Verfahren, beispielsweise durch Destillation, erfolgen.

Bei dem Methylolalkanal der allgemeinen Formel I handelt es sich bevorzugt um Dimethylolalkanal, Pentaerythrose oder Hydroxypivalinaldehyd.

Der Hydrierfeed wird vor dem Hydrierreaktoreingang mit tertiärem Amin vermischt bis der Hydrierfeed einen pH-Wert von 6,3 bis 7,8 aufweist. Es ist auch möglich, den Hydrierfeed und das tertiäre Amin getrennt in den Reaktor einzuspeisen und dort zu vermischen.

Als geeignete tertiäre Amine werden die in DE-A 25 07 461 aufgeführten Amine beispielhaft genannt. Als tertiäre Amine sind Tri-n-C₁-bis C₄-alkylamine bevorzugt und Trimethylamin, Triethylamin, Tri-n-propylamin und Tri-n-butylamin besonders bevorzugt.

Amine sind besonders vorteilhaft zur pH-Einstellung zu verwenden, da sie mit Ameisensäure thermisch zersetzbare Salze bilden, welche nach der Hydrierung wieder gespalten werden können. Somit kann ein Salzanfall vermieden werden und das tertiäre Amin kann in den Prozess rückgeführt werden.

Besonders vorteilhaft ist die Verwendung desselben tertiären Amins im Aldolisierungsprozess zum Methylolalkanal - der Kondensation von höherem Aldehyd und Formaldehyd - und in der Hydrierung. Die Messung des pH-Werts erfolgt mit den bekannten Techniken, vorzugsweise mit einer Glaselektrode und einem pH-Meter.

Erfindungsgemäß verwendbare Katalysatoren sind für Hydrierungen geeignete Katalysatoren, die bevorzugt mindestens ein Metall der Nebengruppe 8 bis 12 des Periodensystems der Elemente wie Fe, Ru, Os, Co, Rh, Ir, Ni, Pd, Pt, Cu, Ag, An, Zn, Cd, Hg, bevorzugt Fe, Co, Ni, Cu, Ru, Pd, Pt, besonders bevorzugt Cu, bevorzugt auf einem üblichen Trägermaterial, besonders bevorzugt auf einem aus den Oxiden des Titans, Zirkoniums, Hafniums, Siliziums und/oder Aluminiums aufweisen. Die Herstellung der erfindungsgemäß verwendbaren Katalysatoren kann nach aus dem Stand der Technik bekannten Verfahren zur Herstellung derartigen Trägerkatalysatoren erfolgen. Bevorzugt verwendet werden können auch Trägerkatalysatoren, die Kupfer auf einem Aluminiumoxid- oder Titandioxid-haltigen Trägermaterial in An- oder Abwesenheit eines oder mehrer der Elemente Magnesium, Barium, Zink oder Chrom aufweist. Derartige Katalysatoren und ihre Herstellung sind aus WO 99/44974 bekannt.

Weiterhin sind kupferhaltige Trägerkatalysatoren wie sie z.B. in WO 95/32171 beschrieben sind und die in EP-A 44 444 und DE 19 57 591 offenbarten Katalysatoren für die erfindungsgemäße Hydrierung geeignet.

Die Hydrierung kann diskontinuierlich oder kontinuierlich z.B. in einem mit einer Katalysatorschüttung gefüllten Reaktorrohr, bei dem die Reaktionslösung über die Katalysatorschüttung z.B. in Riesel- oder Sumpffahrweise, wie in DE-A 19 41 633 oder DE-A 20 40 501 beschrieben, geleitet wird. Es kann vorteilhaft sein, einen Teilstrom des Reaktionsaustrages, gegebenenfalls unter Kühlung, zurückzuführen und wieder über das Katalysatorfestbett zu leiten. Ebenso kann es vorteilhaft sein, die Hydrierung in mehreren hintereinander geschalteten Reaktoren durchzuführen, beispielsweise in 2 bis 4 Reaktoren, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. Dabei kann es zweckmäßig sein, den Hydrieraustrag aus dem vorangehenden Reaktor vor dessen Eintritt in den nachfolgenden Reaktor zu kühlen, beispielsweise mittels Kühlvorrichtungen oder durch Eindüsen kalter Gase, wie Wasserstoff oder Stickstoff oder Einleiten eines Teilstroms an kalter Reaktionslösung.

Die Hydriertemperatur liegt im allgemeinen zwischen 50 und 180°C, bevorzugt 90 und 140°C. Als Hydrierdruck werden im allgemeinen 10 bis 250 bar, bevorzugt 20 bis 120 bar angewandt.

Die Hydrierung kann unter Zugabe eines inerten Lösungsmittels durchgeführt werden. Als Lösungsmittel sind cyclische Ether, wie THF oder Dioxan, als auch acyclische Ether einsetzbar, ebenso niedere Alkohole z.B. Methanol, Ethanol oder 2-Ethylhexanol.

Im übrigen können beliebige Hydriermethoden angewendet und Hydrierkatalysatoren eingesetzt werden, wie sie für die Hydrierung von Aldehyden üblich und in der Standardliteratur eingehend beschrieben sind.

### Beispiel 1

Hydrierung von Hydroxypivalinaldehyd zu Neopentylglykol

### Hydrierfeed

1,1 mol Isobutyraldehyd wurden mit 1 mol Formaldehyd in Form einer 40 %igen Lösung und 4 mol.% Trimethylamin, bezogen auf Isobutyraldehyd, bei 75°C 1 h lang gerührt. Die Reaktionslösung wurde eingeengt, indem bei Normaldruck Leichtsieder wie beispielsweise Isobutyralydehyd und ein Teil des Wassers abdestilliert wurden. Der erhaltene Sumpf bestand aus 75 Gew.-% Hydroxypivalinaldehyd, 20 Gew.-% Wasser und ca. 5 Gew.-% sonstigen organischen Nebenkomponenten.

### Verwendeter Katalysator

Es wurde Katalysator G gemäß WO 95/32171 verwendet.

### Hydrierung

Als Ausgangslösung diente das vorstehend als Hydrierfeed beschriebene Gemisch. Dieses Gemisch wurde durch Zusatz von Trimethylamin jeweils auf den in Tabelle 1 angegebenen pH-Wert eingestellt und in einem Hydrierreaktor mit Flüssigkeitskreislauf (Kreislauf: Zulauf =10:1) mit einer Katalysatorbelastung von 0,3 kg_{HPA}/Iₖₐₜ x h in Rieselfahrweise bei 40 bar und 125°C über den Katalysator gepumpt. In einem Nachreaktor wurde im geraden Durchgang bei 40 bar und 125°C der Umsatz vervollständigt.

Einen Vergleich des erfindungsgemäßen Verfahren mit den Vergleichsbeispielen 1 und 2, bei denen der pH-Wert des Hydrierfeeds jeweils außerhalb des erfindungsgemäßen Bereichs liegt, zeigt Tabelle 1.

Zur pH-Wert-Messung wurde ein pH-Meter der Firma Knick Modell 766 mit einer Glaselektrode N1041A der Firma Schott verwendet.

**Tabelle 1:**

| Bsp. | pH- | HPA² [GC-Gew.-%] | NPG³ [GC-Gew.-%] | i-BuOH⁴ [GC-Gew.-%] | HPN⁵ [GC-Gew.-%] | Acetal⁶ [GC-Gew.-%] | Selektivität |
|---|---|---|---|---|---|---|---|
| V1 | 5,3¹ | 0,03 | 91,97 | 2,63 | 0,92 | 0,43 | 96,71 |
| 1 | 7,4 | 0,02 | 93,04 | 2,10 | 0,90 | 0,11 | 97,83 |
| V2 | 8,3 | 0,07 | 91,89 | 3,08 | 1,04 | 0,09 | 96,62 |
| ¹ ohne Zusatz von Amin | | | | | | | |
| ² HPA= Hydroxypivalinaldehyd | | | | | | | |
| ³ NPG = Neopentylglykol | | | | | | | |
| ⁴ i-BuOH = iso-Butylalkohol | | | | | | | |
| ⁵ HPN = Hydroxypivalinsäureneopentylglykolester | | | | | | | |
| ⁶ Acetal = NPG-Hydroxypivalinaldehyd-Acetal | | | | | | | |

## Patentansprüche

1. Verfahren zur katalytischen Hydrierung von Methylolalkanalen der allgemeinen Formel 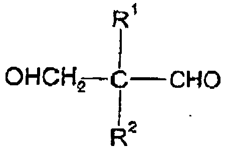
in der R¹ und R² unabhängig voneinander eine weitere Methylolgruppe oder eine Alkylgruppe mit 1 bis 22 C-Atomen oder eine Aryl-oder Aralkylgruppe mit 6 bis 33 C-Atomen bedeuten, in der Flüssigphase an einem Hydrierkatalysator, **dadurch gekennzeichnet, dass** durch Zusatz mindestens eines tertiären Amins im Hydrierfeed ein pH-Wert von 6,3 bis 7,8 eingestellt wird und
dass eine Formaldehyd-arme wässrige Methylolalkanallösung als Hydrierfeed eingesetzt wird, wobei der Gehalt an Formaldehyd unter 5 Gew.-% liegt.

2. Verfahren nach Anspruch 1, **dadurch gekennzeichnet, dass** ein Tri-n-alkylamin verwendet wird.

3. Verfahren nach einem der Ansprüche 1 bis 2, **dadurch gekennzeichnet**, das Trimethylamin, Trimethylamin, Tri-n-propylamin und oder Tri-n-butylamin zugesetzt wird.

4. Verfahren nach einem der Ansprüche 1 bis 3, **dadurch gekennzeichnet, dass** der Hydrierkatalysator mindestens ein Metall der Nebengruppen 8 bis 12 des Periodensystems der Elemente aufweist.

5. Verfahren nach einem der Ansprüche 1 bis 4, **dadurch gekennzeichnet, dass** es sich um einen Trägerkatalysator handelt.

6. Verfahren nach Anspruch 5, **dadurch gekennzeichnet, dass** als Trägermaterial die Oxide des Titans, Zirkoniums, Hafniums, Siliciums und/oder Aluminiums verwendet werden.

7. Verfahren nach einem der Ansprüche 4 bis 6, **dadurch gekennzeichnet, dass** der Hydrierkatalysator Kupfer auf einem Aluminiumoxid- oder Titandioxid-haltigen Trägermaterial in An- oder Abwesenheit eines oder mehrerer der Elemente Magnesium, Barium, Zink oder Chrom aufweist.

8. Verfahren nach einem der Ansprüche 1 bis 7, **dadurch gekennzeichnet, dass** es sich um Hydroxypivalinaldehyd, Pentaerythrose oder Dimethylolbutanal handelt.

## Claims

1. A process for the catalytic hydrogenation of methylolalkanals of the formula 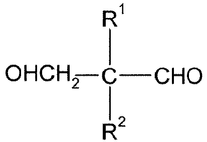
where R¹ and R² are each, independently of one another, a further methylol group or an alkyl group having from 1 to 22 carbon atoms or an aryl or aralkyl group having from 6 to 33 carbon atoms, in the liquid phase over a hydrogention catalyst, wherein the pH of the hydrogenation feed is set to from 6.3 to 7.8 by addition of at least one tertiary amine and
wherein an aqueous methylolalkanal solution which is low in formaldehyde is used as hydrogenation feed, wherein the formaldehyde content is less than 5% by weight.

2. The process according to claim 1, wherein a tri-n-alkylamine is used.

3. The process according to either of claims 1 and 2, wherein trimethylamine, triethylamine, tri-n-propylamine and/or tri-n-butylamine is/are used.

4. The process according to any of claims 1 to 3, wherein the hydrogenation catalyst comprises at least one metal of transition groups 8 to 12 of the Periodic Table of the Elements.

5. The process according to any of claims 1 to 4, wherein the hydrogenation catalyst is a supported catalyst.

6. The process according to claim 5, wherein the oxides of titanium, zirconium, hafnium, silicon and/or aluminum are used as support material.

7. The process according to any of claims 4 to 6, wherein the hydrogenation catalyst comprises copper on an Al₂O₃- or TiO₂-containing support material in the presence or absence of one or more of the elements magnesium, barium, zinc and chromium.

8. The process according to any of claims 1 to 7, wherein the methylolalkanal which is hydrogenated is hydroxypivalaldehyde, pentaerythrose or dimethylolbutanal.

## Revendications

1. Procédé d'hydrogénation catalytique de méthylolalcanals de formule générale : 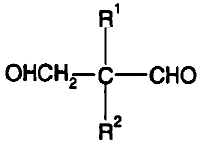
dans laquelle R¹ et R² signifient indépendamment l'un de l'autre un autre groupe méthylol ou un groupe alkyle avec 1 à 22 atomes de carbone ou un groupe aryle ou aralkyle avec 6 à 33 atomes de carbone, en phase liquide sur un catalyseur d'hydrogénation, **caractérisé en ce que** par l'addition d'au moins une amine tertiaire dans le mélange d'alimentation d'hydrogénation, on règle un pH compris entre 6,3 et 7,8 et
**en ce qu'**on utilise une solution de méthylolalcanal aqueuse pauvre en formaldéhyde comme mélange d'alimentation d'hydrogénation, la teneur en formaldéhyde étant inférieure à 5 % en poids.

2. Procédé selon la revendication 1, **caractérisé en ce qu'**on utilise une tri-n-alkylamine.

3. Procédé selon l'une des revendications 1 à 2, **caractérisé en ce qu'**on ajoute la triméthylamine, la triéthylamine, la tri-n-propylamine ou la tri-n-butylamine.

4. Procédé selon l'une des revendications 1 à 3, **caractérisé en ce que** le catalyseur d'hydrogénation présente au moins un métal des sous-groupes 8 à 12 du système périodique des éléments.

5. Procédé selon l'une des revendications 1 à 4, **caractérisé en ce qu'**il s'agit d'un catalyseur supporté.

6. Procédé selon la revendication 5, **caractérisé en ce qu'**on utilise comme matériau de support les oxydes du titane, du zirconium, de l'hafnium, du silicium et/ou de l'aluminium.

7. Procédé selon l'une des revendications 4 à 6, **caractérisé en ce que** le catalyseur d'hydrogénation présente du cuivre sur un matériau de support contenant de l'oxyde d'aluminium ou du dioxyde de titane en présence ou en l'absence d'un ou de plusieurs des éléments magnésium, baryum, zinc ou chrome.

8. Procédé selon l'une des revendications 1 à 7, **caractérisé en ce qu'**il s'agit d'hydroxypivalinaldéhyde, de pentaérythrose ou de diméthylolbutanal.
